# EUROPEAN PATENT SPECIFICATION

(11) **EP 3 216 465 B1**
(45) Date of publication and mention of the grant of the patent: **17.07.2019**
(21) Application number: 17159961.6
(22) Date of filing: 08.03.2017
(51) Int. Cl.: A61L 9/01

(54) **VOLATILE COMPOSITION**
FLÜCHTIGE ZUSAMMENSETZUNG
COMPOSITION VOLATILE

(30) Priority: 09.03.2016 JP 2016045744
(43) Date of publication of application: 13.09.2017
(73) Proprietor: Shin-Etsu Chemical Co., Ltd., Tokyo (JP)
(72) Inventor: YAMAMOTO, Atsushi, Joetsu-shi, Niigata (JP); KITAMURA, Akira, Joetsu-shi, Niigata (JP); NIINOBE, Shingo, Joetsu-shi, Niigata (JP)
(74) Representative: HGF Limited

(56) References cited:
- EP-A1- 2 986 671
- WO-A1-00/59947
- JP-A- H06 207 162
- US-A- 5 741 482

## Description

### BACKGROUND OF THE INVENTION

### 1. Field of the Invention

The present invention relates to a volatile composition.

### 2. Description of the Related Art

There is a conventionally known volatile preparation such as an air freshener, a volatile insect control agent and a volatile fungicide, in which a solution of a volatile substance in a solvent such as water is dissolved or solubilized in an aqueous solvent, or an active ingredient is carried by a volatile solvent.

Among them, for example, the air freshener is widely used in cars as well as in houses. When an air freshener for cars is used in a parked car, for example, under a blazing sun, a large amount of a volatile substance volatilizes in the car because the temperature reaches about 40 to 80°C in a solar irradiation area in the car. The volatilization amount of a volatile preparation depends on the volatilization rate of a volatile substance itself or a volatile solvent as the carrier. For example, in a high temperature condition, the volatilization rate greatly increases, and the volatile substance is rapidly consumed. This causes a problem that a volatile substance volatilizes in an amount far exceeding the required amount in a fixed space.

To suppress a marked increase in the volatilization amount of a volatile substance in such a high temperature condition, there is provided a volatilization-controllable liquid air freshener, in which a thermosensitive polymer causing thermoreversible aggregation or gelation is used in combination with a volatile substance to control the volatilization rate (JP 06-207162A).
WO 2000059947 describes a process for producing methylcelluloses having greater gel strength and US 5,741,482 describes air treatment gel compositions comprising in an aqueous medium, at least one volatile air treatment agent and a polymeric gelling agent consisting essentially of seaweed gum, guar or guar derivatives.

### SUMMARY OF THE INVENTION

However, examples of the thermosensitive polymer of JP 06-207162A such as methyl cellulose, hydroxypropyl methyl cellulose, polyvinyl methyl ether and partially acetylated polyvinyl alcohol have gelation temperatures of about 60°C, and have small gel strengths. Hence, the effect of suppressing the volatilization amount is limited. Although there is a polymer exhibiting thermosensitivity other than methyl cellulose, such a polymer has a thermal gelation temperature region which is greatly variable depending on a coexistent aroma chemical or surfactant so that it is difficult in practical use.

An object of the present invention is to provide a volatile composition having a small difference in volatilization amounts of a volatile substance within a wide temperature range from the normal temperature and enabling the control of the volatilization amount even at a high temperature by suppressing the volatilization amount of the volatile substance and by increasing the gel strength of the volatile composition.

As a result of intensive studies for achieving the object, the inventors of the present invention have found that a volatile composition enabling the suppression of the volatilization amount of a volatile substance can be produced, and have completed the present invention.

In an aspect of the present invention, there is provided a volatile composition comprising: an alkyl cellulose having such a viscosity that a viscosity at 20°C of a 1% by weight aqueous solution of the alkyl cellulose is 4,000 to 11,000 mPa·s as determined by a Brookfield viscometer and having such a storage elastic modulus that a storage elastic modulus G'(65°C) at 65°C of a 1.5% by weight aqueous solution of the alkyl cellulose is 3,000 to 4,500 Pa; a volatile substance selected from the group consisting of an aroma ingredient, an insecticidal ingredient, an insect control ingredient, and an insect repellent ingredient; and a solvent selected from water, alcohol solvents and glycol ether solvents.

According to the present invention, there is provided a volatile composition which suppresses the volatilization amount of a volatile substance, have a small difference in volatilization amounts within a wide temperature range from the normal temperature, and can control the volatilization amount even at a high temperature.

### BRIEF DESCRIPTION OF THE DRAWINGS

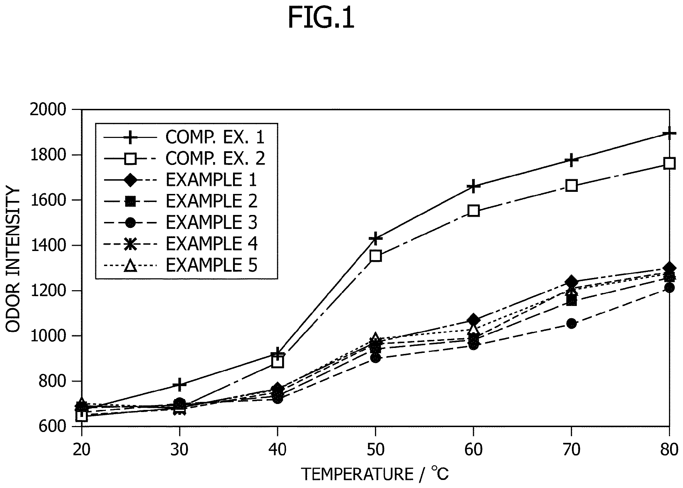

FIG. 1 shows the relation between odor intensity and temperatures as measured in Examples and Comparative examples.

### DETAILED DESCRIPTION OF THE PREFERRED EMBODIMENTS

### (1) Alkyl cellulose

According to the present invention, such an alkyl cellulose that a 1% by weight aqueous solution of the alkyl cellulose has a viscosity at 20°C of 4,000 to 11,000 mPa·s as determined by a Brookfield viscometer, and a 1.5% by weight aqueous solution of the alkyl cellulose has a storage elastic modulus G' (65°C) at 65°C of 3,000 to 4,500 Pa.

The alkyl cellulose can be produced, for example, by a method for producing an alkyl cellulose, comprising the steps of: mixing a cellulose pulp and a first alkali metal hydroxide solution with stirring to obtain alkali cellulose; reacting the alkali cellulose with an alkylating agent to obtain a first reaction mixture; mixing the first reaction mixture and a second alkali metal hydroxide solution with stirring and without further addition of any alkylating agent to obtain a second reaction mixture; and isolate an alkyl cellulose from the second reaction mixture; wherein the ratio of the weight of a first alkali metal hydroxide in the first alkali metal hydroxide solution to the total weight of the first alkali metal hydroxide in the first alkali metal hydroxide solution and a second alkali metal hydroxide in the second alkali metal hydroxide solution is preferably 50 to 86%.

The cellulose pulp includes wood pulp and linter pulp, and is typically used as a raw material of a cellulose ether. The intrinsic viscosity as an index of the polymerization degree of a cellulose pulp can be appropriately selected in accordance with the viscosity of an aqueous solution of an intended cellulose ether, and is preferably 1,000 to 2,200 ml/g, more preferably 1,300 to 2,000 ml/g at 25°C. The intrinsic viscosity of a cellulose pulp can be determined by a method in accordance with method A in JIS P8215.

The cellulose pulp contains cellulose and water. In the present specification, the amount of "the cellulose in a cellulose pulp" can be calculated from the dry matter content determined in accordance with Pulps-Determination of dry matter content in JIS P8203: 1998. The dry matter content is determined by the method comprising the steps of: drying a sample at 105 ± 2°C until the weight reaches a constant value; and calculating the ratio (%) of the weight after drying to the weight before drying as the dry matter content.

The cellulose pulp is preferably a powdered cellulose pulp prepared by pulverization with a pulverizer. The pulp pulverizer may be any pulverizer that can make a cellulose pulp into a powder. Examples of the pulverizer can include a knife mill, a cutting mill, a hammer mill, a ball mill and a vertical roller mill. The cellulose pulp powder preferably has a weight average particle diameter D₅₀ of 30 to 400 µm. The weight average particle diameter D₅₀ of a cellulose pulp powder is determined by the method comprising the steps of: installing a plurality of test sieves having various mesh sizes in accordance with JIS Z8801 in a Ro-Tap® sieve shaker; placing a pulp powder on the uppermost sieve; vibrating or tapping the pulp powder to be sieved; then determining the weight on each sieve and the weight under the sieves to obtain the weight distribution; and determining the average particle diameter at an integrated value of 50% as the weight average particle diameter D₅₀.

Next, the step of mixing a cellulose pulp with a first alkali metal hydroxide solution with stirring to obtain alkali cellulose will be described.

The alkali metal hydroxide solution is divided into a first alkali metal hydroxide solution and a second alkali metal hydroxide solution, and used in two stages. Here, the alkali metal hydroxide solution is not particularly limited, and includes a sodium hydroxide solution and a potassium hydroxide solution. An aqueous sodium hydroxide solution is preferred from the standpoint of economy. A kind of the first alkali metal hydroxide in the first alkali metal hydroxide solution is preferably the same as that of the second alkali metal hydroxide in the second alkali metal hydroxide solution. For example, each of the first and second alkali metal hydroxides is selected to be sodium hydroxide. However, the alkali metal hydroxides can be a combination of different kinds. For example, sodium hydroxide can be used as the first alkali metal hydroxide, while potassium hydroxide can be used as the second alkali metal hydroxide.

As a blending method of the alkali metal hydroxide solution and a cellulose pulp, the alkali metal hydroxide solution is preferably added to a cellulose pulp. Examples of such an addition include direct dropping of the alkali metal hydroxide solution and spraying of the alkali metal hydroxide solution. The spraying is preferred from the standpoint of good uniformity of the resulting alkali cellulose.

The concentration of the alkali metal hydroxide in the alkali metal hydroxide solution is preferably 10 to 60% by weight, more preferably 30 to 50% by weight from the standpoint of etherification efficiency and handleability. The first alkali metal hydroxide and the second alkali metal hydroxide preferably have the same concentrations, but can have different concentrations.

The step of mixing a cellulose pulp and an alkali metal hydroxide solution with stirring is preferably carried out in a reactor having an inner stirring structure. The reactor is preferably equipped with a measurement device such as a device capable of measuring the inside temperature.

Before mixing the cellulose pulp and the first alkali metal hydroxide solution with stirring, it is preferred that oxygen in the reactor be removed by a vacuum pump or the like and be replaced with an inert gas, preferably nitrogen, to suppress depolymerization which can proceed in the presence of an alkali metal hydroxide and oxygen.

Regarding the amount of the first alkali metal hydroxide solution, a molar ratio of the first alkali metal hydroxide to the cellulose in the cellulose pulp (first alkali metal hydroxide/cellulose) is preferably 2.0 to 4.0, more preferably 2.7 to 3.5. When the molar ratio of the first alkali metal hydroxide to the cellulose is less than 2.0, the gelation temperature may be excessively reduced so that viscosity may not be expressed, and an alkyl cellulose having a high gel strength may not be produced. When the molar ratio is more than 4.0, an alkyl cellulose having a high gel strength may not be produced.

The ratio of the weight of the first alkali metal hydroxide in the first alkali metal hydroxide solution to the total weight of the first alkali metal hydroxide in the first alkali metal hydroxide solution and the second alkali metal hydroxide in the second alkali metal hydroxide solution is preferably 50 to 86%, more preferably 65 to 80%, still more preferably 65 to 75%. When the ratio of the weight of the first alkali metal hydroxide to the total weight of the first and second alkali metal hydroxides is less than 50%, the gelation temperature may be reduced so that viscosity may not be expressed, and an alkyl cellulose having a high gel strength may not be produced. When the ratio of the weight of the first alkali metal hydroxide to the total weight of the first and second alkali metal hydroxides is more than 86%, an alkyl cellulose having a high gel strength may not be produced.

The inside temperature of the reactor during blending of the cellulose pulp with the first alkali metal hydroxide, preferably during addition of the first alkali metal hydroxide solution to the cellulose pulp, is preferably 10 to 80°C, more preferably 30 to 70°C from the standpoint of formation of uniform alkali cellulose.

The blending rate of the first alkali metal hydroxide in the first alkali metal hydroxide solution means the molar amount of the first alkali metal hydroxide added per unit time relative to 1 mol of the cellulose pulp, and is preferably 1.5 to 48 [mol/mol·hr], more preferably 4.8 to 18.6 [mol/mol·hr], still more preferably 8 to 15 [mol/mol·hr] from the standpoint of uniformly mixing of the first alkali metal hydroxide solution in the system. After the addition of the first alkali metal hydroxide solution, mixing may be continued with stirring for another 5 to 30 minutes to make the alkali cellulose more uniform.

In order to suppress local heat generation in the reactor, an organic solvent not affecting the alkylation, such as dimethyl ether, can be added to the system before, during, or after the addition of the first alkali metal hydroxide solution.

Next, the produced alkali cellulose is reacted with an alkylating agent to obtain a first reaction mixture.

Examples of the alkylating agent include a methylating agent such as methyl chloride, dimethyl sulfate and methyl iodide; and an ethylating agent such as ethyl chloride, diethyl sulfate and ethyl iodide. Methyl chloride and ethyl chloride are preferred from the standpoint of the solubility of the resulting alkyl cellulose in water and economy.

The inside temperature of the reactor when the alkylating agent is reacted is preferably 40 to 90°C, more preferably 50 to 80°C, from the standpoint of reaction control.

Regarding a molar amount of the alkylating agent to be blended, the ratio of the molar amount of the alkylating agent to the total molar amount of the first and second alkali metal hydroxides (alkylating agent/total alkali metal hydroxide) is preferably 0.8 to 1.5, more preferably 1.0 to 1.3. When the molar ratio (alkylating agent/total alkali metal hydroxide) is less than 0.8, an intended number of alkyl groups may not be substituted. When the molar ratio is more than 1.5, the blending of the excess amount of alkylating agent may lead to an economic disadvantage.

As for the method of blending the alkylating agent, the alkylating agent is preferably added to the alkali cellulose. The period of time for adding the alkylating agent is preferably 30 to 120 minutes, more preferably 40 to 90 minutes from the standpoint of reaction control and productivity.

The alkyl cellulose in the first reaction mixture preferably has a degree of substitution (DS) of alkyl group of 0.75 to 1.68, more preferably 0.81 to 1.68, still more preferably 0.99 to 1.37 from the standpoint of obtaining a desirable viscosity or storage elastic modulus. The degree of substitution (DS) means the average number of hydroxy groups substituted by alkyl groups per glucose ring unit of cellulose.

Subsequently, the alkylated first reaction mixture is mixed with a second alkali metal hydroxide solution with stirring and without further addition of any alkylating agent so that a second reaction mixture is obtained.

The timing of blending the second alkali metal hydroxide solution with the first reaction mixture, in other words, the timing of start of the blending of the second alkali metal hydroxide solution, is preferably after the completion of the addition of 80% by weight or more of the total amount of the alkylating agent to be added, more preferably after the completion of the addition of the total amount of the alkylating agent to be added. When the timing of starting the addition of the second alkali metal hydroxide solution is before the completion of the addition of 80% by weight of the total amount of the alkylating agent to be added, alkyl cellulose having a high gel strength may not be produced.

Regarding the amount of the second alkali metal hydroxide in the second alkali metal hydroxide solution, the molar ratio of the second alkali metal hydroxide to the cellulose in the cellulose pulp (second alkali metal hydroxide/cellulose) is preferably 0.65 to 2.0, more preferably 0.88 to 1.48. When the molar ratio (alkali metal hydroxide/cellulose) is less than 0.65, an alkyl cellulose having a high gel strength may not be produced. When the molar ratio is more than 2.0, the gelation temperature may be excessively reduced so that viscosity may not be expressed, and an alkyl cellulose having a high gel strength may not be produced.

The inside temperature of the reactor at the start of blending of the second alkali metal hydroxide solution with the first reaction mixture, preferably at the start of the addition of the second alkali metal hydroxide solution to the first reaction mixture, is preferably 65 to 90°C, more preferably 75 to 85°C. When the inside temperature of the reactor at the start of the addition of the second alkali metal hydroxide solution is less than 65°C, an alkyl cellulose having a high gel strength may not be produced. When the inside temperature of the reactor at the start of the addition is more than 90°C, heat generation due to mercerization by the alkali metal hydroxide or an exothermic reaction of alkylation may not be controlled. The inside temperature of the reactor at the completion of the blending of the second alkali metal hydroxide solution is preferably 80°C to 100°C, more preferably 85 to 95°C, from the standpoint of production of an alkyl cellulose having a high gel strength,. The temperature at the start of the addition may be lower than the temperature at the completion of the addition, and the temperature difference may be preferably 3 to 20°C, more preferably 4 to 15°C.

The blending rate of the second alkali metal hydroxide in the second alkali metal hydroxide solution means the molar amount of the second alkali metal hydroxide to be blended with the first reaction mixture per unit time relative to 1 mol of the cellulose in the cellulose pulp, and is preferably 0.5 to 9.6 [mol/mol·hr], more preferably 1.0 to 6.5 [mol/mol·hr], still more preferably 1.0 to 3.5 [mol/mol·hr]. When the blending rate of the second alkali metal hydroxide is less than 0.5 [mol/mol·hr], the period of time for blending the second alkali metal hydroxide becomes long so that the reaction time may be extended and an alkyl cellulose having a high gel strength may not be produced. When the blending rate of the second alkali metal hydroxide is more than 9.6 [mol/mol·hr], an alkyl cellulose having a high gel strength may not be produced.

In the step of blending the second alkali metal hydroxide solution with the first reaction mixture, it is preferred that the second alkali metal hydroxide solution be blended while the inside temperature of the reactor be increased at a constant rate from the start to the completion of the blending of the second alkali metal hydroxide solution from the standpoint of production of alkyl cellulose having a high gel strength. The temperature increase rate is preferably 3.0 to 50°C/hr, more preferably 8.0 to 45°C/hr, still more preferably 8.0 to 30°C/hr.

Generally, the alkali cellulose formed by mixing a cellulose pulp with an alkali metal hydroxide solution is etherified with an alkylating agent to produce an alkyl cellulose. In this case, the alkylating agent in the reaction system is gradually consumed as the etherification proceeds. When the inside temperature of the reactor is constant, the reaction rate of the etherification gradually decreases as the alkylating agent is consumed in the reaction system. On this account, by blending the second alkali metal hydroxide solution while increasing the inside temperature of the reactor at a constant rate, the reduction of the reaction rate of the etherification caused by the consumption of the alkylating agent in the reaction system is suppressed, and the reaction rate of the etherification associated with the blending of the second alkali metal hydroxide solution is relatively increased. As a result, an alkyl cellulose having a high viscosity and a high gel strength can be produced.

After the blending of the second alkali metal hydroxide solution, mixing is preferably continued with stirring to complete the etherification.

The inside temperature of the reactor during the mixing with stirring after the blending of the second alkali metal hydroxide solution is preferably 80 to 120°C, more preferably 85 to 100°C, from the standpoint of reaction controllability. In order to complete the reaction, the mixture is preferably heated after the blending of the second alkali metal hydroxide solution.

The period of time for the mixing with stirring after the blending of the second alkali metal hydroxide solution is preferably 10 to 60 minutes, more preferably 20 to 40 minutes from the standpoint of productivity.

An alkyl cellulose can be isolated from the obtained second reaction mixture in the same manner as the usual purification of a crude alkyl cellulose. The method and the device used for the purification are not particularly limited. The purification can be carried out preferably with water, more preferably with hot water (preferably at 60 to 100°C), in consideration of cost efficiency. Specifically, the purification can be carried out by the method comprising the steps of: mixing the second reaction mixture with water in a stirring container, while dissolving the salts generated as by-products; and subjecting the suspension discharged from the stirring container to a separation operation to remove the salts.

After the purification, the product may be optionally dried. The method and the device used for the drying are not particularly limited. The temperature of the alkyl cellulose during the drying is preferably 40 to 80°C.

The produced alkyl cellulose can be optionally pulverized with a common pulverizer such as a ball mill, a roller mill and an impact grinder and then classified through sieves to adjust the particle size.

Examples of the alkyl cellulose produced in this method preferably include methyl cellulose and ethyl cellulose. Methyl cellulose is preferred particularly from the standpoint of suppression of the volatilization amount of a volatile substance as well as a low gelation temperature.

The alkyl cellulose has a degree of substitution (DS) of alkyl group of preferably 1.61 to 2.03, more preferably 1.74 to 2.03. When an alkyl cellulose has a degree of substitution of alkyl group of less than 1.61, the alkyl cellulose may not have high gel strength. When an alkyl cellulose having a degree of substitution of more than 2.03 is produced, large amounts of an alkylating agent and an alkali metal hydroxide are required to be added so that an economic disadvantage may be brought.

Generally, the DS means the degree of substitution and is the average number of hydroxy groups substituted by methoxy groups or ethoxy groups per glucose ring unit of the cellulose.

The degree of substitution of alkyl group of an alkyl cellulose can be determined by the Zeisel-GC method described in J.G. Gobler, E.P. Samscl and G.H. Beaber, Talanta, 9, 474 (1962).

The viscosity at 20°C of a 1% by weight aqueous solution of the alkyl cellulose determined by a Brookfield viscometer is 4,000 to 11,000 mPa·s (the viscosity of a 2% by weight aqueous solution thereof determined by a Brookfield viscometer is 60,000 to 150,000 mPa·s), preferably 4,000 to 8,000 mPa·s (the viscosity of the 2% by weight aqueous solution thereof determined by a Brookfield viscometer is preferably 60,000 to 110,000 mPa·s), still more preferably 4,000 to 7,500 mPa·s (the viscosity of the 2% by weight aqueous solution thereof determined by a Brookfield viscometer is still more preferably 60,000 to 100,000 mPa·s). When the viscosity of the 1% by weight aqueous solution is less than 4,000 mPa·s, a volatile composition comprising the alkyl cellulose has a low viscosity so that sufficient volatilization suppression cannot be achieved. When the viscosity of the 1% by weight aqueous solution is more than 11,000 mPa·s, although depending on the amount of the alkyl cellulose relative to the amount of the volatile composition, a volatile composition has an excessively high viscosity so that the volatile composition is difficult to pour into a container or the like.

The viscosity by a Brookfield viscometer can be determined by the analytical method for methyl cellulose in the Japanese Pharmacopoeia Sixteenth Edition.

The gel strength of an alkyl cellulose is represented by the storage elastic modulus G'(65°C) at 65°C of a 1.5% by weight aqueous solution thereof. Generally, the storage elastic modulus means an elastic factor of a solution, or a factor of the characteristics that the deformation caused by a force applied to a substance is returned to the original shape when the force is removed, and is an index of gel strength.

The storage elastic modulus G'(65°C) at 65°C of a 1.5% by weight aqueous solution of the alkyl cellulose is 3,000 to 4,500 Pa, preferably 3,300 to 4,500 Pa, more preferably 3,300 to 4,300 Pa. When the storage elastic modulus
G'(65°C) is less than 3,000 Pa, a volatile composition has a low gel strength so that the volatilization amount of a volatile active ingredient may not be suppressed.

The storage elastic modulus G'(65°C) of a 1.5% by weight aqueous solution of an alkyl cellulose may be determined with a rheometer such as MCR 500 manufactured by Anton Paar.

The 1.5% by weight aqueous solution of an alkyl cellulose is prepared by the following method comprising the steps of: placing an exact amount of an alkyl cellulose corresponding to 4.50 g of the dried alkyl cellulose in a wide-mouthed bottle, which is a container having a diameter of 65 mm, a height of 120 mm and a volume of 350 ml; adding hot water (98°C) to the bottle to make a total amount to be 300.0 g; putting a lid on the bottle; then stirring the mixture with a stirrer at 350 to 450 rpm for 20 minutes until a uniform dispersion liquid is obtained; and stirring the resulting liquid in a water bath at 5°C or less for 40 minutes for dissolution to obtain a sample solution.

The temperature of the sample-measuring section of a rheometer is adjusted to 65°C in advance; the prepared 1.5% by weight aqueous solution of an alkyl cellulose is poured into a CC27 measurement cup, which is a cylindrical aluminum container having a diameter of 30 mm and a height of 80 mm, to a marked line of 25 ml; and the angular frequency is selected to be 1 rad/s, and a distortion with a vibration amplitude of 10% is applied with a bob cylinder (with a diameter of 26.7 mm and a height of 40.0 mm: CC27) to start the measurement. The temperature of the measuring section is maintained constantly at 65°C. The data are collected at one point per minute. The maximum storage elastic modulus from the start to elapsed time of 60 minutes in the measurement is regarded as the storage elastic modulus G'(65°C) in the present invention.

The gelation temperature of an alkyl cellulose is evaluated by using the relation between a storage elastic modulus G'(30→80°C) and a loss elastic modulus G". Generally, the loss elastic modulus means a viscous factor of a solution, or a factor of the characteristics that a resistance is generated by deformation of a fluid with fluid movement, and is an index of gelation temperature.

The gelation temperature of a 1.5% by weight aqueous solution of the alkyl cellulose is preferably 40 to 55°C, more preferably 44 to 53°C, still more preferably 48 to 53°C. When the gelation temperature is less than 40°C, such an alkyl cellulose may have an excessively low dissolution temperature in water so that the alkyl cellulose may not be dissolved and may fail to express sufficient viscosity. When the gelation temperature is more than 55°C, a volatile composition comprising the alkyl cellulose may have low gel strength so that the volatilization amount of a volatile active ingredient may not be suppressed.

The gelation temperature of a 1.5% by weight aqueous solution of an alkyl cellulose may be determined with a rheometer such as MCR 500 manufactured by Anton Paar.

The 1.5% by weight aqueous solution of an alkyl cellulose is prepared in the same method as that for preparing the sample solution for the storage elastic modulus G'(65°C).

The temperature of the sample-measuring section of a rheometer is adjusted to 30°C in advance; the 1.5% by weight aqueous solution of an alkyl cellulose is poured into a CC27 measurement cup, which is a cylindrical container having a diameter of 30 mm and a height of 80 mm, to a marked line of 25 ml; and the frequency is selected to be 1 Hz, and a distortion with a vibration amplitude of 0.5% is applied to start the measurement. The temperature of the sample-measuring section is increased by 2°C per minute to 80°C. The data are collected at two points per minute.

The storage elastic modulus G'(30→80°C) and the loss elastic modulus G" determined by the measurement are variable as the temperature of a measurement system increases. The temperature at which the loss elastic modulus G" becomes equal to the storage elastic modulus G'(30→80°C), that is, the temperature at which G"/G'(30→80°C) becomes a value of one, is regarded as the gelation temperature.

The alkyl cellulose is a polymer having the characteristics of causing thermoreversible aggregation or gelation when heated in a dissolved state in water, and is hydrophilic at low temperatures, but loses the hydrophilicity or becomes hydrophobic at high temperatures. In particular, the methyl cellulose is known to have a comparatively small interaction in a wide range with an active ingredient of an aroma chemical or the like, or with a surfactant or the like. This is considered to be because, for example, methyl cellulose dissolved in a solvent has such a chain structure as to restrict the free rotation of the methyl cellulose itself, and thus the interaction with an active ingredient of an aroma chemical or the like, or with a surfactant or the like is restricted.

The content of the alkyl cellulose in the volatile composition is preferably 0.1 to 20% by weight, more preferably 0.5 to 10% by weight.

### (2) Volatile substance

The volatile substance is selected from an aroma ingredient, an insecticidal ingredient, an insect control ingredient, and an insect repellent ingredient. Examples of the aroma ingredient include vanillin, orange oil, α-pinene, limonene, ethyl formate and linalyl benzoate. Examples of the insecticidal ingredient or the insect control ingredient include p-dichlorobenzene, naphthaline, terallethrin, allethrin and prallethrin. Examples of the insect repellent ingredient include caryophyllene, eugenol, methylchavicol and methyl cinnamate. The content of the volatile substance in the volatile composition is preferably 0.01 to 40% by weight, more preferably 0.5 to 10% by weight.

### (3) Solvent

The solvent is selected from water and alcohol solvents and glycol ether solvents. For example, from the standpoint of vapor pressure, safety, melting point and less solvent odor, preferred are lower alcohol solvents such as methanol, ethanol, 1-propanol and 2-propanol; and glycol ether solvents such as 3-methoxy-3-methyl-1-butanol, 3-methoxy-1-butanol, propylene glycol monomethyl ether, propylene glycol monoethyl ether, propylene glycol monobutyl ether, ethylene glycol monomethyl ether, ethylene glycol monoethyl ether and ethylene glycol monobutyl ether. The solvent can be used singly or in a combination of two or more.

The content of the solvent in the volatile composition is preferably 40 to 95% by weight, more preferably 60 to 90% by weight.

### (4) Others

The volatile composition may comprise an optional gelling agent. Examples of the gelling agent include agar, proteoglycan, glycoprotein, pectic acid, pectinic acid, alginic acid, carrageenan, gellan gum, guar gum, xanthan gum, locust bean gum, pectin, gelatin, casein, starch, galactomannan, carboxymethyl cellulose, hydroxypropyl cellulose, hydroxypropyl methyl cellulose and polyacrylic acids. The gelling agent can be used singly or in a combination of two or more.

The content of the gelling agent in the volatile composition is preferably 0.1 to 30% by weight, more preferably 1 to 10% by weight.

The volatile composition may further comprise an optional inorganic salt, an optional solubilizing substance, or an optional emulsifying substance. The content of the salt or each substance in the volatile composition is preferably 0.5 to 20% by weight, more preferably 1 to 10% by weight.

The inorganic salt is exemplified by sodium chloride and magnesium sulfate, and can control the temperature of aggregation or gelation.

Examples of the solubilizing substance include nonionic surfactants such as polyoxyethylene alkyl ethers, polyoxyethylene alkyl phenyl ethers, polyoxyethylene styryl phenyl ethers, polyoxyethylene-polyoxypropylene glycols, polyhydric alcohol fatty acid partial esters, polyoxyethylene fatty acid esters, polyoxyethylenated castor oils and polyoxyethylene alkylamines; and anionic surfactants such as fatty acid salts, alkylbenzene sulfonates, alkyl sulfonates, dialkyl sulfosuccinates, alkylsulfates, polyoxyethylene alkyl ether sulfates and alkyl phosphates. When the volatile composition is prepared in a solution form, such a solubilizing substance may be added to help the composition to be dissolved.

Examples of the emulsifying substance include said surfactants, casein, lecithin, gum arabic, cationic surfactants, glycols and lanolin.

The volatile composition may be prepared as a solution form by dissolving the alkyl cellulose in an aqueous solvent such as water or in a volatile solvent, or may be prepared as a gel form by directly adding the alkyl cellulose to an aqueous composition containing a volatile ingredient or to a volatile solvent.

In a preferred embodiment of the liquid volatile composition, the volatile composition is placed in a container and allows a volatile ingredient to be volatilized through micropores or capillaries present in paper, fibers, a nonwoven fabric, a welded resin product, ceramic, a sintered metal or the like. The volatilization suppression mechanism in this case is considered to be as follows: An alkyl cellulose aggregates or gels as the temperature increases, and adheres to the micropores or capillaries to prevent the liquid containing a volatile substance from moving and volatilizing.

In a preferred embodiment of the gel volatile composition, the volatile composition is placed in an open-mouth container such as an open-mouth container of glass, plastic or metal, and allows a volatile ingredient to be volatilized through the open mouth without the micropores or capillaries. The volatilization suppression mechanism in this case is considered to be as follows: An alkyl cellulose aggregates or gels as the temperature increases, so that a film-like structure is formed on the surface of the volatile composition, or the viscosity of the whole system is increased, thereby making it difficult for a volatile ingredient in the volatile composition to move to the surface.

### EXAMPLES

Synthesis Examples and Comparative Synthesis Examples of methyl cellulose will be presented next, and the present invention will be further explained in detail with reference to Examples and Comparative Examples. It should not be construed that the invention is limited to or by Synthesis Examples and Examples.

### <Synthesis Example 1>

A wood pulp having an intrinsic viscosity of 1,350 ml/g was pulverized by a pulverizer to obtain a cellulose pulp powder. Of the cellulose pulp powder, the cellulose pulp powder in an amount corresponding to 6.0 kg of cellulose component was placed in an internal-stirring pressure-resistant reactor with a jacket. Nitrogen substitution was carried out by using evacuation to thoroughly remove oxygen in the reactor.

Next, the reactor was stirred while adjusting the inside temperature of the reactor to 60°C. A 49% by weight aqueous sodium hydroxide solution as a first alkali metal hydroxide solution was added to the cellulose at an addition rate of 10.48 [mol/mol·hr]. As a result, a molar ratio of a first sodium hydroxide to the cellulose (first sodium hydroxide/cellulose) became 2.62, and alkali cellulose was obtained.

Subsequently, 2.4 kg of dimethyl ether was added, and the inside temperature of the reactor was controlled so as to maintain the inside temperature of the reactor at 60°C. After the addition of dimethyl ether, methyl chloride was added over 60 minutes, while increasing the inside temperature of the reactor from 60°C to 80°C. As a result, a molar ratio of the amount of methyl chloride to the total amount of the first and the later second sodium hydroxides (methyl chloride/total sodium hydroxide) became 1.1, and a first reaction mixture was obtained. After the addition of methyl chloride, a 49% by weight aqueous sodium hydroxide solution as a second alkali metal hydroxide solution was added at an addition rate of 3.20 [mol/mol·hr]. As a result, a molar ratio of a second sodium hydroxide to the cellulose (second sodium hydroxide/cellulose) became 1.60, and a second reaction mixture was obtained. The inside temperature of the reactor was 77°C at the start of the addition of the second sodium hydroxide solution, and 89°C at the completion of the addition. The inside temperature of the reactor was increased at 24°C/hr from the start to the completion of the addition of the second aqueous sodium hydroxide solution. After the completion of the addition of the second aqueous sodium hydroxide solution, the stirring was continued for 30 minutes to complete the etherification. The ratio of the weight of the first sodium hydroxide in the first aqueous sodium hydroxide solution to the total weight of the first and second sodium hydroxides in the first and second aqueous sodium hydroxide solutions was 62.1%.

The obtained second reaction mixture was made into a slurry by adding hot water of 95°C, was then washed with a rotary pressure filter, and was dried with an air dryer. The dried product was pulverized with a ball mill and classified through sieves to obtain methyl cellulose.

The obtained methyl cellulose had a DS of 1.81, and the viscosity at 20°C of a 1% by weight aqueous solution thereof determined by a Brookfield viscometer was 4,300 mPa·s (the viscosity at 20°C of a 2% by weight aqueous solution thereof determined by a Brookfield viscometer was 59,000 mPa·s). The storage elastic modulus G'(65°C) at 65°C of a 1.5% by weight aqueous solution of the methyl cellulose was determined to be 3,000 Pa, and the gelation temperature was 48°C. The obtained results are shown in Table 1.

### <Synthesis Example 2>

The same procedure as in Synthesis Example 1 was carried out to obtain methyl cellulose except that a cellulose pulp powder prepared by pulverizing a wood pulp having an intrinsic viscosity of 1,600 ml/g with a pulverizer was used.

The obtained methyl cellulose had a DS of 1.82, and the viscosity at 20°C of a 1% by weight aqueous solution thereof determined by a Brookfield viscometer was 7,200 mPa·s (the viscosity at 20°C of a 2% by weight aqueous solution thereof determined by a Brookfield viscometer was 99,000 mPa·s). The storage elastic modulus G'(65°C) at 65°C of a 1.5% by weight aqueous solution of the methyl cellulose was determined to be 3,500 Pa, and the gelation temperature was 46°C. The obtained results are shown in Table 1.

### <Synthesis Example 3>

The same procedure as in Synthesis Example 1 was carried out to obtain methyl cellulose except that a cellulose pulp powder prepared by pulverizing a wood pulp having an intrinsic viscosity of 2,000 ml/g with a pulverizer was used.

The obtained methyl cellulose had a DS of 1.83, and the viscosity at 20°C of a 1% by weight aqueous solution thereof determined by a Brookfield viscometer was 11,000 mPa·s (the viscosity at 20°C of a 2% by weight aqueous solution thereof determined by a Brookfield viscometer was 150,000 mPa·s). The storage elastic modulus G'(65°C) at 65°C of a 1.5% by weight aqueous solution of the methyl cellulose was determined to be 4,500 Pa, and the gelation temperature was 50°C. The obtained results are shown in Table 1.

### <Synthesis Example 4>

A cellulose pulp was placed in a reactor in the same manner as in Synthesis Example 1 except that a cellulose pulp powder prepared by pulverizing a wood pulp having an intrinsic viscosity of 1,400 ml/g with a pulverizer was used. The reactor was stirred while adjusting the inside temperature of the reactor to 55°C. A 49% by weight aqueous sodium hydroxide solution as a first alkali metal hydroxide solution was added thereto at an addition rate of 12.04 [mol/mol·hr]. As a result, a molar ratio of a first sodium hydroxide to the cellulose (first sodium hydroxide/cellulose) became 3.01, and alkali cellulose was obtained.

Subsequently, the same procedure as in Synthesis Example 1 was carried out to obtain a first reaction mixture. Next, the same procedure as in Synthesis Example 1 was carried out to obtain a second reaction mixture except that the inside temperature of the reactor was 81°C at the start of the addition of the second aqueous sodium hydroxide solution, the inside temperature of the reactor was 89°C at the completion of the addition, the inside temperature of the reactor was increased at 16.4°C/hr from the start to completion of the addition of the second aqueous sodium hydroxide solution, the second aqueous sodium hydroxide solution was added at an addition rate of 2.58 [mol/mol·hr], and as a result, a molar ratio of the second sodium hydroxide to the cellulose (second sodium hydroxide/cellulose) became 1.26. The ratio of the weight of the first sodium hydroxide in the first aqueous sodium hydroxide solution to the total weight of the first and second sodium hydroxides in the first and second aqueous sodium hydroxide solutions was 70.5%.

The obtained second reaction mixture was then purified and pulverized in the same manner as in Synthesis Example 1, and methyl cellulose was obtained. The experimental conditions are shown in Table 1.

The obtained methyl cellulose had a DS of 1.85, and the viscosity at 20°C of a 1% by weight aqueous solution thereof determined by a Brookfield viscometer was 6,000 mPa·s (the viscosity at 20°C of a 2% by weight aqueous solution thereof determined by a Brookfield viscometer was 82,000 mPa·s). The storage elastic modulus G'(65°C) at 65°C of a 1.5% by weight aqueous solution of the methyl cellulose was determined to be 3,300 Pa, and the gelation temperature was 53°C. The obtained results are shown in Table 1.

### <Synthesis Example 5>

In the same manner as in Synthesis Example 4, a cellulose pulp was placed in a reactor. The reactor was stirred while adjusting the temperature of the reactor to 55°C. A 49% by weight aqueous sodium hydroxide solution as a first alkali metal hydroxide solution was added thereto at an addition rate of 11.39 [mol/mol·hr]. As a result, a molar ratio of a first sodium hydroxide to the cellulose (first sodium hydroxide/cellulose) became 2.85, and alkali cellulose was obtained.

Subsequently, the same procedure as in Synthesis Example 1 was carried out to obtain a first reaction mixture. Next, the same procedure as in Synthesis Example 1 was carried out to obtain a second reaction mixture except that the inside temperature of the reactor was 79°C at the start of the addition of the second aqueous sodium hydroxide solution and 91°C at the completion of the addition, the inside temperature of the reactor was increased at 24°C/hr from the start to the completion of the addition of the second aqueous sodium hydroxide solution, the second aqueous sodium hydroxide solution was added at an addition rate of 2.80 [mol/mol·hr], and as a result, a molar ratio of the second sodium hydroxide and the cellulose (second sodium hydroxide/cellulose) became 1.40. The ratio of the weight of the first sodium hydroxide in the first aqueous sodium hydroxide solution to the total weight of the first and second sodium hydroxides in the first and second aqueous sodium hydroxide solutions was 67.0%.

The obtained second reaction mixture was then purified and pulverized in the same manner as in Synthesis Example 1 to obtain methyl cellulose. The experimental conditions are shown in Table 1.

The obtained methyl cellulose had a DS of 1.82, and the viscosity at 20°C of a 1% by weight aqueous solution thereof determined by a Brookfield viscometer was 6,050 mPa·s (the viscosity at 20°C of a 2% by weight aqueous solution thereof determined by a Brookfield viscometer was 82,500 mPa·s). The storage elastic modulus G'(65°C) at 65°C of a 1.5% by weight aqueous solution of the methyl cellulose was determined to be 3,300 Pa, and the gelation temperature was 51°C. The obtained results are shown in Table 1.

**Table 1**

| | production conditions | | | | | | | | properties | | | |
|---|---|---|---|---|---|---|---|---|---|---|---|---|
| | addition of methyl chloride | a first NaOH | | | a second NaOH | | | | degree of substitution of methoxy (DS) | viscosiy of 1 wt% aq. solution determined by Brookfield viscometer | storage elastic modulus G'(65°C) of 1.5 wt% aq. solution | gelation temperature of 1.5wt% aq. solution |
| | | weight ratio of first NaOH to total amount of first and second NaOH | molar ratio of first NaOH to cellulose | addtion rate of first NaOH to cellulose | molar ratio of second NaOH to cellulose | addition rate of second NaOH to cellulose | inside temp. of reactor at start of addition | temp. increase rate | | | | |
| | | (%) | | (mol/mol·hr) | | (mol/mol·hr) | (°C) | (°C/hr) | | (mPa·s) | (Pa) | (°C) |
| Syn. Ex. 1 | one stage | 62.1 | 2.62 | 10.48 | 1.60 | 3.20 | 77 | 24 | 1.81 | 4,300 | 3,000 | 48 |
| Syn. Ex .2 | one stage | 62.1 | 2.62 | 10.48 | 1.60 | 3.20 | 77 | 24 | 1.82 | 7,200 | 3,500 | 46 |
| Syn. Ex.3 | one stage | 62.1 | 2.62 | 10.48 | 1.60 | 3.20 | 77 | 24 | 1.83 | 11,000 | 4,500 | 50 |
| Syn. Ex.4 | one stage | 70.5 | 3.01 | 12.04 | 1.26 | 2.58 | 81 | 16.4 | 1.85 | 6,000 | 3,300 | 53 |
| Syn. Ex .5 | one stage | 67.0 | 2.85 | 11.39 | 1.40 | 2.80 | 79 | 24 | 1.82 | 6,050 | 3,300 | 51 |

### <Example 1>

In the same method as the preparation method of the sample solution for storage elastic modulus G'(65°C), a 1.5% by weight aqueous solution of the methyl cellulose obtained in Synthesis Example 1 was prepared.

The 100 g of the obtained aqueous solution of the methyl cellulose and 0.5 g of vanilla essence (manufactured by Meidi-Ya Store) were placed and mixed in a 100-ml beaker to obtain a volatile composition having a volatile substance concentration of 0.5% by weight.

The 40 g of the volatile composition was placed in an alcohol lamp having a volumetric size of 70 ml. The temperature of the alcohol lamp was controlled in a constant-temperature water bath, and the odor intensity was quantitatively determined from 20 to 80°C. The odor intensity was measured by using a portable odor sensor XP-329IIIR manufactured by New Cosmos Electric Co., Ltd. As for the measurement method, the alcohol lamp was allowed to stand in each constant temperature bath of 20 to 80°C for 30 minutes, then the sensor part of a portable odor sensor was placed at a position 1 cm apart from the cap of the alcohol lamp, and the odor intensity after 30 seconds was recorded. The measurement results of the odor intensity at the respective temperatures are shown in FIG. 1.

### <Example 2>

The same procedure as in Example 1 was carried out to obtain a volatile composition except that the methyl cellulose obtained in Synthesis Example 2 was used. In the same manner as in Example 1, a portable odor sensor was used to determine the odor intensity at temperatures from 20 to 80°C. The results are shown in FIG. 1.

### <Example 3>

The same procedure as in Example 1 was carried out to obtain a volatile composition except that the methyl cellulose obtained in Synthesis Example 3 was used. In the same manner as in Example 1, a portable odor sensor was used to determine the odor intensity at temperatures from 20 to 80°C. The results are shown in FIG. 1.

### <Example 4>

The same procedure as in Example 1 was carried out to obtain a volatile composition except that the methyl cellulose obtained in Synthesis Example 4 was used. In the same manner as in Example 1, a portable odor sensor was used to determine the odor intensity at temperatures from 20 to 80°C. The results are shown in FIG. 1.

### <Example 5>

The same procedure as in Example 1 was carried out to obtain a volatile composition except that the methyl cellulose obtained in Synthesis Example 5 was used. In the same manner as in Example 1, a portable odor sensor was used to determine the odor intensity at temperatures from 20 to 80°C. The results are shown in FIG. 1.

### <Comparative Example 1>

The same procedure as in Example 1 was carried out to obtain a volatile composition except that 100 g of distilled water was used in the place of the methyl cellulose. In the same manner as in Example 1, a portable odor sensor was used to determine the odor intensity at temperatures from 20 to 80°C. The results are shown in FIG. 1.

### <Comparative Example 2>

The same procedure as in Example 1 was carried out to obtain a volatile composition except that methyl cellulose SM-15 manufactured by Shin-Etsu Chemical Co., Ltd. and described in JP 06-207162A was used. In the same manner as in Example 1, a portable odor sensor was used to determine the odor intensity at temperatures from 20 to 80°C. The results are shown in FIG. 1.

The volatile compositions of Examples 1 to 5 suppressed the increase of the odor intensity when heated at 40°C or more, so that the volatilization amount was able to be controlled. In contrast, the volatile compositions of Comparative Examples 1 and 2 increased the odor intensity at 40°C or more, and the volatilization amount was not able to be controlled. It is evident from the results that when the volatile compositions of Examples 1 to 5 are used, for example, as an air freshener for cars in an environment in which the temperature in a parked car under a blazing sun reaches about 40 to 80°C, the volatile compositions can control the volatilization amount and can reduce the difference in the volatilization amounts within a wide temperature range from the normal temperature.

## Claims

1. A volatile composition comprising:
an alkyl cellulose having such a viscosity that a viscosity at 20°C of a 1% by weight aqueous solution of the alkyl cellulose is 4,000 to 11,000 mPa·s as determined by a Brookfield viscometer and having such a storage elastic modulus that a storage elastic modulus G'(65°C) at 65°C of a 1.5% by weight aqueous solution of the alkyl cellulose is 3,000 to 4,500 Pa;
a volatile substance selected from the group consisting of an aroma ingredient, an insecticidal ingredient, an insect control ingredient, and an insect repellent ingredient; and
a solvent selected from water, alcohol solvents and glycol ether solvents.

2. The volatile composition according to claim 1, wherein a 1.5% by weight aqueous solution of the alkyl cellulose has a gelation temperature of 40 to 55°C.

3. The volatile composition according to claim 1 or claim 2, wherein the alkyl cellulose is methyl cellulose having a degree of substitution (DS) of alkyl group of 1.61 to 2.03.

4. The volatile composition according to any one of claims 1 to 3, further comprising a gelling agent.

## Patentansprüche

1. Flüchtige Zusammensetzung, umfassend:
eine Alkylcellulose, die eine solche Viskosität aufweist, dass eine Viskosität bei 20 °C von einer wässrigen Lösung von 1 Gewichtsprozent der Alkylcellulose 4000 bis 11.000 mPa·s ist, bestimmt durch ein Brookfield-Viskosimeter, und ein solches elastisches Speichermodul aufweist, dass ein elastisches Speichermodul G' (65 °C) bei 65 °C einer wässrigen Lösung von 1,5 Gewichtsprozent der Alkylcellulose 3000 bis 4500 Pa ist;
eine flüchtige Substanz, ausgewählt aus der Gruppe bestehend aus einem Aromabestandteil, einem insektiziden Bestandteil, einem Insektenbekämpfungsbestandteil und einem Insektenschutzmittelbestandteil; und
ein Lösungsmittel, ausgewählt aus Wasser, Alkohollösungsmitteln und Glykoletherlösungsmitteln.

2. Flüchtige Zusammensetzung nach Anspruch 1, wobei eine wässrige Lösung von 1,5 Gewichtsprozent der Alkylcellulose eine Geliertemperatur von 40 bis 55 °C aufweist.

3. Flüchtige Zusammensetzung nach Anspruch 1 oder Anspruch 2, wobei die Alkylcellulose Methylcellulose mit einem Substitutionsgrad (degree of substitution, DS) der Alkylgruppe von 1,61 bis 2,03 ist.

4. Flüchtige Zusammensetzung nach einem der Ansprüche 1 bis 3, ferner umfassend ein Geliermittel.

## Revendications

1. Composition volatile comprenant :
une alkyl-cellulose ayant une viscosité telle qu'une viscosité à 20° C d'une solution aqueuse de 1 % en poids de l'alkyl-cellulose soit de 4 000 à 11 000 mPa comme déterminé par un viscosimètre Brookfield et ayant un module élastique de conservation tel qu'un module élastique de conservation G' (65° C) à 65° C d'une solution aqueuse de 1,5 % en poids de l'alkyl-cellulose soit de 3 000 à 4 500 Pa ;
une substance volatile sélectionnée dans le groupe constitué d'un ingrédient aromatique, un ingrédient insecticide, un ingrédient anti-insecte et un ingrédient répulsif d'insecte ;
et un solvant sélectionné parmi l'eau, des solvants d'alcool et des solvants d'éther de glycol.

2. Composition volatile selon la revendication 1, dans laquelle une solution aqueuse de 1,5 % en poids de l'alkyl-cellulose a une température de gélification de 40 à 55° C.

3. Composition volatile selon la revendication 1 ou 2, dans laquelle l'alkyl-cellulose est une méthylcellulose ayant un degré de substitution (DS) du groupe alkyl de 1,61 à 2,03.

4. Composition volatile selon l'une quelconque des revendications 1 à 3, comprenant en outre un agent de gélification.
